# EUROPÄISCHE PATENTSCHRIFT

(11) **EP 0 942 692 B1**
(45) Veröffentlichungstag und Bekanntmachung des Hinweises auf die Patenterteilung: **01.09.2004**
(21) Anmeldenummer: 97950194.7
(22) Anmeldetag: 14.11.1997
(51) Int. Cl.: A61F 2/38

(54) **FEMORALES TEIL EINER KNIEGELENKPROTHESE**
FEMUR PART OF A KNEE JOINT PROSTHESIS
PARTIE FEMORALE D'UNE PROTHESE DE L'ARTICULATION DU GENOU

(30) Priorität: 14.11.1996 DE 19647155
(43) Veröffentlichungstag der Anmeldung: 22.09.1999
(73) Patentinhaber: PLUS ENDOPROTHETIK AG, 6343 Rotkreuz (CH)
(72) Erfinder: SCHMOTZER, Hans, CH-5000 Aarau (CH)
(74) Vertreter: Popp, Eugen, Dr.
(86) Internationale Anmeldenummer: PCT/EP1997/006377
(87) Internationale Veröffentlichungsnummer: WO 1998/020817

(56) Entgegenhaltungen:
- EP-A- 0 384 854
- US-A- 5 201 768

## Beschreibung

Die Erfindung betrifft ein Implantat nach dem Oberbegriff des Anspruchs 1.

Ein Implantat der eingangs genannten Art ist aus der EP-A-0 384 854 bekannt. Ferner ist in der DE 42 30 118 C2 ein Implantat beschrieben welches zumindest einen Teil der Kondylenfläche bei einem Femurknochen ersetzen Kann. Dazu weist es eine Femurschale auf, die mittels eines Zapfens am Knochen befestigbar ist. Die Femurschale sitzt auf einer vorbereiteten Plateaufläche des Knochens auf.

Um einen sicheren Halt des Implantats am Knochen gewährleisten zu können, muß sichergestellt sein, daß ein zum Befestigen des Implantats verwendeter Knochenzement eine möglichst große Verbindungsfläche schafft und in alle Hohlräume eindringt. Um dies auch bei leichtem Andrücken des Implantats sicherzustellen und zu vermeiden, daß der Knochenzement oder ein fließfähiges Knochenmaterial nicht seitlich über die Schale herausquillt, ist an dieser randständig eine unterbrechungslos zirkulär umlaufende, gewölbeartig konkave Ausformung vorgesehen.

Weitere, ähnliche Implantate sind in den EP 0 709 075 A1, EP 0 611 559 A1 und WO 95/24874 beschrieben.

Allerdings ist bei allen vorgenannten Implantaten folgendes Problem nicht gelöst. Bei gestrecktem Gelenk sollte die belastete Gelenkauflagefläche zwischen dem Implantat und der zugeordneten Gelenkpfanne möglichst groß sein, damit eine kleine Flächenpressung und eine gute Stabilität gewährleistet ist. Durch eine kleine Flächenpressung wird unter anderem eine schnelle Abnutzung des künstlichen Gelenkteils verhindert.

Eine große Auflagefläche wird erreicht, indem man einen Krümmungsradius der Gelenkfläche in diesem Bereich groß bzw. dessen Krümmung klein wählt.

Die gewünschte großflächige Auflage bei gestrecktem Gelenk bringt jedoch folgenden Nachteil mit sich. Wird das Implantat schräg eingesetzt, so daß seine Längsausrichtung nicht mit der Schwenkebene des Gelenkes übereinstimmt, so kann es beim Abbiegen des Gelenkes zu einer Kantenpressung kommen. Durch eine solche Kantenpressung werden sowohl die Gelenkpfanne als auch das Implantat überbeansprucht und sehr schnell abgenutzt.

Aufgabe der Erfindung ist es, ein Implantat der eingangs genannten Art dahingehend weiterzubilden, daß bei gestrecktem Gelenk die Flächenpressung der Gelenkauflagefläche des Implantats gering gehalten ist, jedoch auch bei nicht exakt ausgerichtetem Einbau desselben eine Kantenpressung bzw. falsche Belastung des Gelenkes beim Abbiegen verhindert werden kann.

Diese Aufgabe wird durch die im Anspruch 1 genannten Merkmale gelöst.

Demgemäß weist die kufenförmige Schale eine Gelenkauflagefläche auf, deren Krümmungsradius quer zu ihrer Längserstreckung zumindest im coronalen Bereich größer ist als deren Krümmungsradius quer zu ihrer Längserstreckung in einem Bereich, der bei abgebogenem Gelenk belastet ist, wobei in einem dazwischen befindlichen Übergangsbereich der größere Krümmungsradius kontinuierlich in den kleineren Krümmungsradius übergeht.

Durch den großen Krümmungsradius der Gelenkauflagefläche im coronalen Bereich, ist eine große Auflagefläche des Implantats in der Gelenkpfanne bei gestrecktem Gelenk sichergestellt. Eine übermäßige Abnutzung wird verhindert und eine gute Stabilität des Gelenkes gewährleistet.

Wird das Gelenk abgebogen, so verändert sich der Krümmungsradius der Gelenkauflagefläche an der in der Gelenkpfanne aufliegenden Stelle kontinuierlich und zwar geht der große Krümmungsradius in den kleineren Krümmungsradius über. Dadurch kann auch bei nicht exakt eingesetztem Implantat eine Kantenpressung beim Abbiegen des Gelenkes vermieden werden. Darüber hinaus kann die Bewegungsfreiheit für das jeweilige Gelenk, beispielsweise zur Seite hin oder bezüglich einer Rotation, erhöht werden.

Eine besonders bevorzugte Ausführungsform ist dadurch gekennzeichnet, daß der Übergangsbereich sich über einen Winkelbereich von etwa 30° bis 70°, insbesondere 40° bis 60° erstreckt. Dabei sollte der Übergangsbereich nicht in den coronalen Bereich hineinreichen, da dadurch an dieser Stelle eine geringere Auflagefläche zustandekommen würde. Ferner sollte die Gelenkauflagefläche bei vollständig abgebogenem Gelenk mit dem kleinsten Radius in der Gelenkpfanne zur Auflage kommen. Vorzugsweise beginnt der Übergangsbereich beispielsweise bei einem Implantat zum Ersetzen eines Teils einer Kondylenfläche bei einem Femurknochen etwa im dorsalen Bereich der kufenförmigen Schale oder etwas versetzt in Richtung des coronalen Bereiches.

Gemäß einer bevorzugten Ausführungsform ist die kufenförmige Schale an einem Ende, welches der belasteten Auflagefläche bei abgebogenem Gelenk abgewandt ist, schiffsbugartig zugespitzt ausgebildet.

Zur besseren Verankerung des Implantats im Knochen ist knochenseitig (beispielsweise proximalseitig) vorzugsweise zumindest ein Zapfen oder dgl. Mittel angeordnet. Ebenso kann knochenseitig sich in Längsrichtung, von dorsal nach ventral erstreckend eine Mittelrippe vorgesehen sein. Diese Mittelrippe wird in einem komplementären Schlitz im Knochen aufgenommen. Diese Maßnahme dient zur besonders exakten Ausrichtung des Implantats gegenüber dem Knochen und auch zur Stabilisierung.

Gemäß einer besonders bevorzugten Ausführungsform besitzt die kufenförmige Schale einen zumindest teilweise zirkulär umlaufenden Rand, wobei zwischen den Rändern selbst oder zwischen den Rändern einerseits und der Mittelrippe andererseits eine oder mehrere Vertiefungen, Ausnehmungen oder Taschen vorgesehen sind. In diese Ausnehmungen kann der Knochenzement eindringen, so daß er beim Aufpressen des Implantats auf den Knochen nicht über deren Ränder hervorquillt. Ferner kann der Knochenzement durch geeignete Ausbildung dieser Taschen oder Ausnehmungen beim Anbringen des Implantats in alle Hohlräume eingebracht werden. Die Mittelrippe kann an den Enden bis zum jeweiligen Umfangsrand der Schale hochgezogen sein.

Wird ein Implantat nur für eine Kondylenfläche benötigt, so kann es aus nur einer kufenförmigen Schale bestehen, die die entsprechende Kondylenfläche ersetzt. In diesem Falle handelt es sich bei dem Implantat um einen unikondylären Schlitten.

Sollten die Kondylenflächen von zwei benachbarten Kondylen mit Implantatteilen ersetzt werden, so können zwei zumindest im wesentlichen parallele kufenförmige Schalen einstückig oder miteinander verbunden beim Implantat vorgesehen sein. Dann handelt es sich bei dem Implantat um einen bikondylären Schlitten.

Die Erfindung wird nachfolgend - auch in Hinblick auf weitere Merkmale und Vorteile - mit Bezug auf die beiliegenden Zeichnungen erläutert. Die Zeichnungen zeigen in
- Fig. 1: eine schematischen Seitenansicht eines Ausführungsbeispiels eines erfindungsgemäßen Implantats in Form eines unikondylären Schlittens,
- Fig. 2: eine schematischen Vorderansicht des unkondylären Schlittens aus Fig. 1,
- Fig. 3: eine schematischen Draufsicht des unikondylären Schlittens aus Fig. 1,
- Fig. 4 und 5: jeweils schematische Perspektivansichten des unikondylären Schlittens aus Fig. 1,
- Fig. 6: eine Schnittansicht des unikondylären Schlittens aus Fig. 1 mit Schnitt gemäß Linie B-B in Fig. 1 und
- Fig. 7: eine Schnittansicht des unikondylären Schlittens aus Fig. 1 mit Schnitt gemäß Linie A-A in Fig. 1.

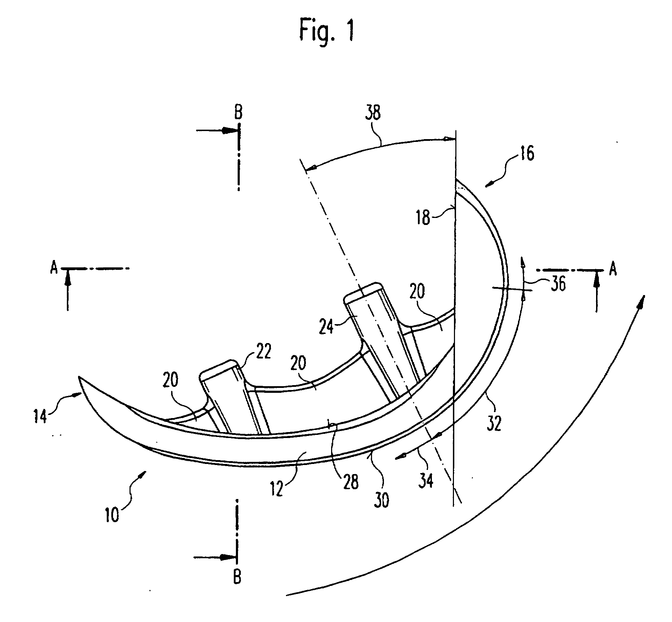

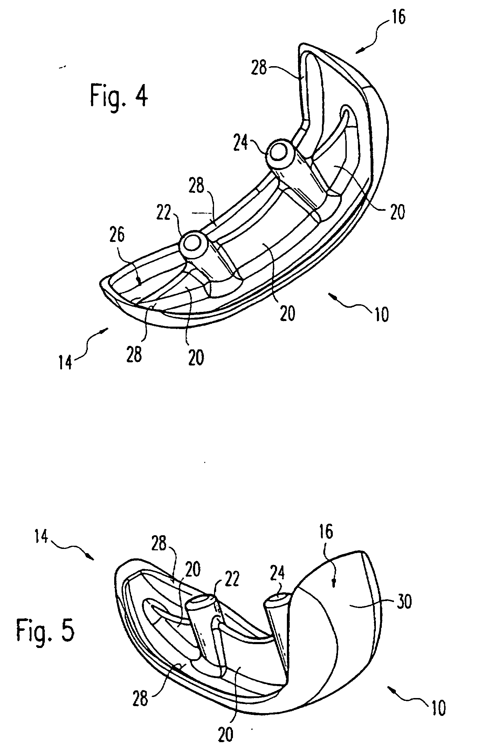

In den Fig. 1 bis 7 ist eine Ausführungsform eines Implantats 10 zum Ersetzen eines Teils der Kondylenfläche eines Femurknochens in Form eines unikondylären Schlittens dargestellt.

Das Implantat 10 umfaßt eine kufenförmig ausgebildete Schale 12 mit einem ventralen 14 und eine dorsalen 16 Ende, wobei eine dorsale Schnittfläche 18 am dorsalen Ende 16 im spitzen Winkel (Doppelpfeil 38) zu den Achsen von (später noch beschriebenen) Zapfen 22, 24 liegt.

Proximalseitig ist an der am Gelenkknochen (nicht dargestellt) zu befestigenden Seite eine sich vom ventralen 14 zum dorsalen 16 Ende erstreckende, in Längsrichtung verlaufende Mittelrippe 20 vorgesehen, in die die zwei in Längsrichtung voneinander beabstandeten, im wesentlichen parallelen Zapfen 22 und 24 integriert sind. Durch die Zapfen 22, 24 ist die Mittelrippe 20 in drei Teile gegliedert. Alternativ können natürlich auch nur ein oder mehr als zwei Zapfen vorgesehen werden.

Die Zapfen 22, 24 dienen im wesentlichen zur stabilen Befestigung des Implantats am Knochen, wozu ein Knochenzement, beispielsweise autologes/homologes Knochenmaterial, verwendet wird. Da die Zapfen 22, 24 in einer jeweils komplementären Bohrung im Knochen geführt sein können, dienen sie zusätzlich der korrekten Ausrichtung des Implantats 10.

Zur zusätzlichen Stabilisierung aber auch zur korrekten Ausrichtung dient ebenfalls die Mittelrippe 20, die in einer entsprechend ausgeformten Nut (nicht dargestellt) im Knochen aufgenommen und geführt wird. Bei exakt ausgebildeter Nut im Knochen ist die Längsrichtung des Implantats 10 genau in der Schwenkebene des Gelenkes ausgerichtet.

In dem beschriebenen Ausführungsbeispiel ist die Mittelrippe 20 an beiden Enden jeweils nicht ganz bis zu den Umfangsrändern 28 der Schale 12 hochgezogen. Alternativ kann die Mittelrippe 20 auch ganz hochgezogen werden.

Durch die Mittelrippe 20 ist zusammen mit dem zirkulär umlaufenden, hochgezogenen Umfangsrand 28 der Schale 12 eine zumindest teilweise umlaufende Ausnehmung bzw. Tasche 26 gebildet, in welcher der Knochenzement beim Andrücken des Implantats an den Knochen aufgenommen wird und sich möglichst in alle Hohlräume zwischen Implantat und Knochen hineinverteilt. Der umlaufende Umfangsrand 28 ist im vorliegenden Beispiel nicht als Schneidkante ausgeführt.

Am ventralen Ende 14 ist die kufenförmige Schale 12 schiffsbugartig ausgebildet. Aber auch ein andersartig geformtes Ende ist wählbar.

An der distalen Seite der kufenförmigen Schale 12 erstreckt sich eine Gelenkauflagefläche 30 in Längsrichtung der kufenartigen Schale 12. Die Gelenkauflagefläche 30 eines fertig eingesetzten Implantats 10 gleitet in einer nicht dargestellten Gelenkpfanne, die beispielsweise mit Polyethylen beschichtet ist.

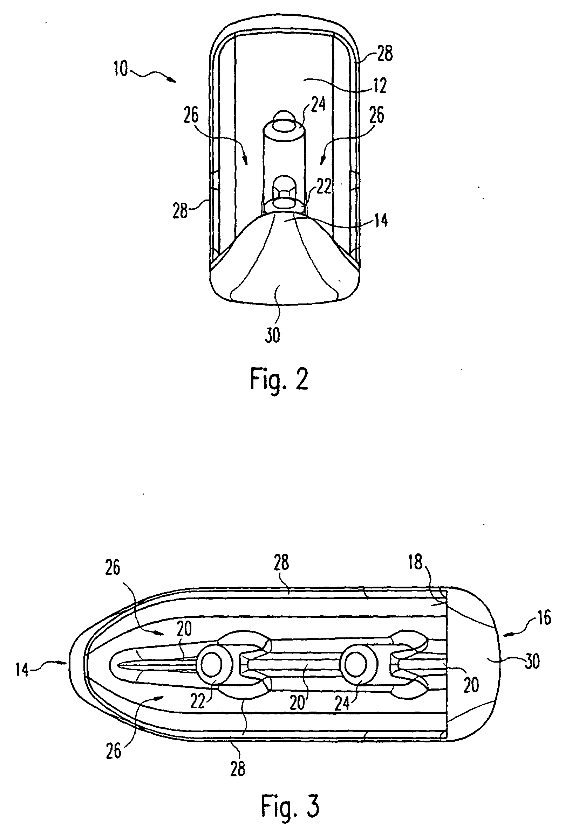

Die Gelenkauflagefläche 30 weist quer zu ihrer Längserstrekkung an jeder Stelle eine bestimmte Krümmung mit einem entsprechenden Krümmungsradius auf und ist an den parallel zur Längsrichtung verlaufenden Umfangsrändern 28 nach oben gezogen, wie insbesondere in Fig. 2 gut zu erkennen ist, so daß die kufenförmige Schale 12 entsteht.

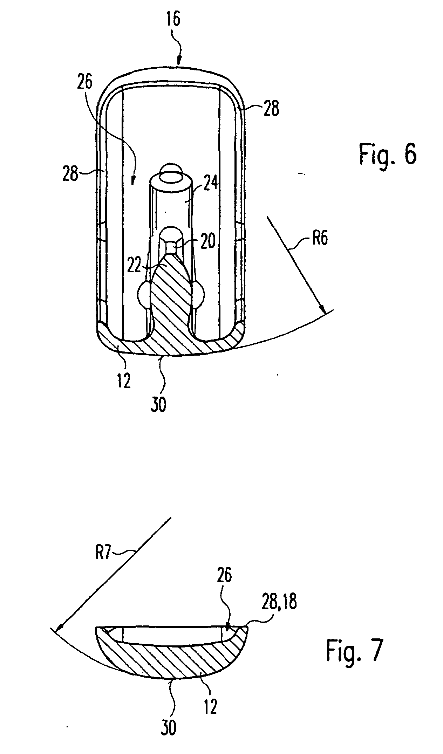

Gemäß Fig. 6 (Schnitt B-B aus Fig. 1 in der coronalen Ebene) ist der Krümmungsradius R₆ im Coronalbereich groß gewählt, d.h. die Krümmung ist relativ flach. Dadurch wird eine große Auflagefläche zwischen Implantat und der komplementären Lagerfläche der zugeordneten Gelenkpfanne sichergestellt, was zur Stabilität und geringen Abnutzung des Gelenkes beiträgt.

Ist das Implantat jedoch etwas schräg zur Schwenkachse des Gelenkes eingesetzt, so kann es bei dem oben beschriebenen Krümmungsradius beim Abbiegen des Gelenkes zu einer Kantenpressung kommen. Als Folge davon würde sich das Gelenk nicht nur schnell abnutzen. Es könnten auch Fehlbelastungen der Knochen und Muskulatur durch die entstehenden Kräfte auftreten.

Zur Vermeidung dieses Effekts wird im dorsalen Bereich der Auflagefläche ein kleiner Krümmungsradius R₇ gewählt (also R₆ > R₇); d.h. eine große Krümmung.

Zwischen den beiden Bereichen mit den Radien R₆ und R₇ ist ein Übergangsbereich 32 (vgl. Fig. 1) vorgesehen, in dem die beiden Radien R₆ und R₇ kontinuierlich ineinander übergehen.

Der Übergangsbereich 32 beim vorliegenden Ausführungsbeispiel erstreckt sich von etwas unterhalb der Dorsalebene bis etwa zur Achse des Zapfens 24. Von da an bis zum ventralen Ende ist die Gelenkfläche mit dem Radius R₆ ausgeführt (Pfeil 34).

Vom anderen Ende des Übergangsbereichs 32 in dorsaler Richtung besitzt die Gelenkauflagefläche 30 den Krümmungsradius R₇ (Pfeil 36).

Insgesamt kann beim Abbiegen eines nicht ganz exakt eingefügten Implantats 10 die kufenförmige Schale 12 ohne Behinderung in der Gelenkpfanne gleiten. Eine Kantenpressung wird vermieden. Dennoch kann bei gestrecktem Gelenk eine große Auflagefläche sichergestellt werden.

Natürlich können auch zwei kufenförmige Schalen 12 in einem Implantat zumindest im wesentlichen parallel zueinander vorgesehen sein. Dann würde es sich um eine bikondylären Schlitten handeln, mit dem zwei nebeneinander angeordnete Kondylenflächen ersetzt werden könnten. Dazu könnten die beiden kufenförmigen Schalen einfach einteilig in einem Implantat ausgeformt sein oder aber durch ein Verbindungsteil miteinander verbunden werden.

Es sollte klar sein, daß die Erfindung nicht auf die vorliegende Ausführungsform beschränkt ist. Die Erfindung kann auf jedes Implantat angewendet werden, das im Gelenkbereich eingesetzt wird, in dem ein Verschwenkvorgang stattfindet. In dem Zustand, in dem das Gelenk meist am stärksten belastet ist, soll eine große Auflagefläche sichergestellt werden, also eine kleine Krümmung bzw. ein großer Krümmungsradius der Gelenkauflagefläche.

Dagegen soll beim Abbiegen des Gelenkes keine Kantenpressung auftreten, so daß in Verschwenk- oder Abbiegerichtung der Krümmungsradius der Gelenkauflagefläche abnehmen bzw. die Krümmung entsprechend zunehmen soll.

### Bezugszeichenliste

- 10: Implantat (hier: unikondylärer Schlitten)
- 12: kufenförmige Schale
- 14: ventrales Ende
- 16: dorsales Ende
- 18: dorsale Schnittfläche
- 20: Mittelrippe
- 22: Zapfen
- 24: Zapfen
- 26: Ausnehmung, Tasche
- 28: umlaufender Umfangsrand
- 30: Gelenkauflagefläche
- 32: Übergangsbereich
- 34: Pfeil R₆
- 36: Pfeil R₇
- 38: spitzer Winkel

## Patentansprüche

1. Implantat zur Anordnung auf einer Plateau- oder Resektionsfläche eines Gelenkknochens umfassend zumindest eine kufenförmige Schale (12) mit einem ventralen (14) und einem dorsalen (16) Ende, welche kufenförmige Schale (12) eine sich in Längsrichtung erstreckende Gelenkauflagefläche (30) besitzt, deren Krümmungsradius (R₆) quer zu ihrer Längserstreckung zumindest im coronaten, bei gestrecktem Gelenk belasteten Bereich größer ist als ihr Krümmungsradius (R₇) quer zu ihrer Längserstreckung in einem Bereich, der bei vollständig abgebogenem Gelenk belastet ist, wobei in einem dazwischen befindlichen Übergangsbereich (32) der größere Krümmungsradius (R₆) in den kleineren Krümmungsradius (R₇) übergeht,
**dadurch gekennzeichnet, daß** die Gelenkauflagefläche (30) vom einen Ende des Übergangsbereichs (32) bis zum ventrelen Ende der Gelenkauflagefläche mit dem größeren Krümmungsradius (R₆) und vom anderen Ende des Übergangsbereichs in dorsaler Richtung mit dem kleineren, Krümmungsradius (R₇) ausgebildet ist, und daß der Übergang vom größeren Krümmungsradius (R₆) in den kleineren Krümmungsradius (R₇) kontinuierlich ist und sich der Übergangsbereich (32) über einen Winkelbereich von 40° bis 60° erstreckt.

2. Implantat nach Anspruch 1,
**dadurch gekennzeichnet, daß**
der Übergangsbereich (32) im wesentlichen in dem Bereich der kufenförmigen Schale (12) beginnt, der bei vollständig abgebogenem Gelenk belastet ist, oder in einem Bereich, der geringfügig in Richtung des coronalen Bereiches hin verschoben ist.

3. Implantat nach Anspruch 1 oder 2,
**dadurch gekennzeichnet, daß**
die kufenförmige Schale (12) an dem Ende (14), welches der belasteten Gelenkauflagefläche bei vollständig abgebogenem Gelenk abgewandt ist, schiffsbugartig zugespitzt ausgebildet ist.

4. Implantat nach einem der Ansprüche 1 bis 3,
**dadurch gekennzeichnet, daß**
die kufenförmige Schale (12) knochenseitig zumindest einen Zapfen (22, 24) oder dgl. Mittel aufweist, der oder die zur Verankerung und/oder Ausrichtung in den Knochen einführbar und in diesem befestigbar sind.

5. Implantat nach einem der Ansprüche 1 bis 4,
**dadurch gekennzeichnet, daß**
knochenseitig der kufenförmigen Schale (12) eine sich zumindest im wesentlichen von ventral nach dorsal erstreckende, also in Längsrichtung verlaufende Mittelrippe (20) vorgesehen ist.

6. Implantat nach einem der Ansprüche 1 bis 5,
**dadurch gekennzeichnet, daß**
die kufenförmige Schale (12) einen zirkulär umlaufenden Umfangsrand (28) besitzt, wobei zwischen den Umfangsrändern (28) selbst oder den Umfangsrändern (28) einerseits und der Mittelrippe (20) andererseits eine oder mehrere Vertiefungen, Ausnehmungen oder Taschen (26) angeordnet sind.

7. Implantat nach einem der Ansprüche 1 bis 6 mit einer einzigen kufenförmigen Schale (12), die als unikondylärer Schlitten dient.

8. Implantat nach einem der Ansprüche 1 bis 6 mit zwei zumindest im wesentlichen parallelen kufenförmigen Schalen, die einen bikondylären Schlitten bilden.

## Claims

1. An implant for arrangement on a plateau or resection surface of a joint bone, comprising at least one runner-shaped shell (12) having a ventral (14) and a dorsal (16) end, the runner-shaped shell (12) having a longitudinally extending joint-supporting surface (30), of which the radius of curvature (R₆) transversely to its longitudinal extent, at least in the coronal region which is loaded when the joint is extended, is greater than its radius of curvature (R₇) transversely to its longitudinal extent in a region which is loaded when the joint is fully bent, wherein, in a transition region (32) lying therebetween, the larger radius of curvature (R₆) merges into the smaller radius of curvature (R₇), **characterised in that** the joint-supporting surface (30) is formed with the larger radius of curvature (R₆) from one end of the transition region (32) as far as the ventral end of the joint-supporting surface and with the smaller radius of curvature (R₇) from the other end of the transition region towards the dorsal end, and **in that** the transition from the larger radius of curvature (R₆) to the smaller radius of curvature (R₇) is continuous and the transition region (32) extends over an angular range of 40° to 60°.

2. An implant according to claim 1, **characterised in that** the transition region (32) begins substantially **in that** region of the runner-shaped shell (12) which is loaded when the joint is fully bent, or in a region which is shifted slightly towards the coronal region.

3. An implant according to claim 1 or 2, **characterised in that** the runner-shaped shell (12) is tapered in the shape of a ship's bow at the end (14) remote from the loaded joint-supporting surface when the joint is fully bent.

4. An implant according to any one of claims 1 to 3, **characterised in that** the runner-shaped shell (12) has, on the bone side, at least one pin (22, 24) or similar means insertable into the bone and fixable therein for anchorage and/or alignment.

5. An implant according to any one of claims 1 to 4, **characterised in that** a central rib (20) is provided on the bone side of the runner-shaped shell (12) and extends at least substantially in a ventral-dorsal direction, i.e. longitudinally.

6. An implant according to any one of claims 1 to 5, **characterised in that** the runner-shaped shell (12) has a circularly extending circumferential edge (28), one or more depressions, recesses or pockets (26) being arranged between the circumferential edges (28) themselves or between the circumferential edges (28) on one side and the central rib (20) on the other side.

7. An implant according to any one of claims 1 to 6, comprising a single runner-shaped shell (12) serving as a unicondylar component.

8. An implant according to any one of claims 1 to 6, comprising two at least substantially parallel runner-shaped shells forming a bicondylar component.

## Revendications

1. Implant destiné à être placé sur une surface de plateau ou surface de résection d'un os d'une articulation, comprenant au moins une coquille (12) en forme de patin avec une extrémité ventrale (14) et une extrémité dorsale (16), laquelle coquille (12) en forme de patin présente une surface d'appui d'articulation (30) s'étendant dans la direction longitudinale dont le rayon de courbure (R₆) est supérieur, transversalement à son étendue longitudinale, au moins dans la zone coronale, sollicitée lorsque l'articulation est tendue, à son rayon de courbure (R₇) transversalement à son étendue longitudinale dans une zone qui est sollicitée lorsque l'articulation est entièrement repliée, le grand rayon de courbure (R₆) se prolongeant par le petit rayon de courbure (R₇) dans une zone de transition (32) se trouvant entre les deux premières,
**caractérisé en ce que** la surface d'appui d'articulation (30) est réalisée avec le plus grand rayon de courbure (R₆) d'une extrémité de la zone de transition (32) à l'extrémité ventrale de la surface de support d'articulation, et avec le plus petit rayon de courbure (R₇) de l'autre extrémité de la zone de transition dans la direction dorsale, et **en ce que** la transition du plus grand rayon de courbure (R₆) au plus petit rayon de courbure (R₇) est continue et la zone de transition (32) s'étend sur une plage angulaire de 40° à 60°.

2. Implant selon la revendication 1,
**caractérisé en ce que**
la zone de transition (32) commence sensiblement dans la zone de la coquille (12) en forme de patin qui est sollicitée lorsque l'articulation est totalement repliée, ou dans une zone qui est légèrement déplacée en direction de la zone coronale.

3. Implant selon la revendication 1 ou 2,
**caractérisé en ce que**
la coquille (12) en forme de patin est réalisée pointue à la manière d'une proue de bateau à l'extrémité (14) qui est tournée à l'opposé de la surface de support d'articulation sollicitée lorsque l'articulation est totalement repliée.

4. Implant selon l'une des revendications 1 à 3,
**caractérisé en ce que**
la coquille (12) en forme de patin comporte côté os au moins une tige (22, 24) ou un moyen similaire qui peut être introduit dans l'os pour l'ancrage et/ou l'orientation et qui peut être fixé dans celui-ci.

5. Implant selon l'une des revendications 1 à 4,
**caractérisé en ce que**
côté os de la coquille (12) en forme de patin, il est prévu une nervure centrale (20) s'étendant au moins sensiblement du côté ventral vers le côté dorsal, c'est-à-dire dans la direction longitudinale.

6. Implant selon l'une des revendications 1 à 5,
**caractérisé en ce que**
la coquille (12) en forme de patin présente un bord périphérique (28) s'étendant en cercle, un ou plusieurs renfoncements, évidements ou poches (26) étant disposés entre les bords périphériques (28) eux-mêmes ou les bords périphériques (28) d'une part et la nervure centrale (20) d'autre part.

7. Implant selon l'une des revendications 1 à 6 avec une seule coquille (12) en forme de patin qui sert aussi de prothèse à glissement à un seul condyle.

8. Implant selon l'une des revendications 1 à 6 avec deux coquilles en forme de patin au moins sensiblement parallèles qui forment une prothèse à glissement à deux condyles.
